# EUROPEAN PATENT SPECIFICATION

(11) **EP 1 217 374 B1**
(45) Date of publication and mention of the grant of the patent: **01.06.2005**
(21) Application number: 01830729.8
(22) Date of filing: 28.11.2001
(51) Int. Cl.: G01N 33/52

(54) **Method of determining the iodine content in biological fluids, and semiautomatic apparatus for carrying out the same**
Verfahren und semiautomatisches Gerät zur Bestimmung des Jodinhaltes in biologischen Flüssigkeiten
Méthode et appareil semiautomatiques pour la détermination de la teneur d'iode dans fluides biologiques

(30) Priority: 22.12.2000 IT RM000693
(43) Date of publication of application: 26.06.2002
(73) Proprietor: Ente per le Nuove Tecnologie, l'Energia e l'Ambiente - ENEA, 00196 Rome (IT)
(72) Inventor: Sedda, Antioco Franco, 00192 Roma (IT); Rossi, Gabriele, 00069 Trevignano (RM) (IT); Cipriani, Cesidio, 00185 Roma (IT)
(74) Representative: Sarpi, Maurizio

(56) References cited:
- EP-A- 1 037 044
- US-A- 5 137 690

## Description

The present invention relates to biological and clinical analysis, and in particular a method and the relative apparatus to carry out the analysis of the so-called "iodide content", i.e. the iodine content in the human organism, which is particularly used in nuclear medicine and endocrinology as well.

The determination of the iodine content in biological fluids, and particularly in urine, is the most important method of finding out alimentary and/or pathological deficiencies or an excess of iodine in the organism. The analytical measure of the iodide content in urine is of particular importance also in the therapy of thyroid tumors where a destruction of the thyroid tissues by selective radiation with ¹³¹I (radio ablation) is made.

Since the presence of an important iodine content in the patient, as better shown thereafter, inhibits the effects of the radio ablation, it is necessary to carry out an accurate analytical screening of iodide (ion I⁻) in urine not only of patients having a deficiency in the iodine intake but also before the therapy with ¹³¹I to find out and correct any pathological and/or accidental overloading.

At the present state of art, the determination of I⁻ in urine is made normally in chemical laboratories according to reliable analytical methods that need, however, a complex analytical instrumentation which is not frequently used in the hospital wards.

It should be noted that all of the iodine passing to blood after its ingestion together with water or food under several chemical forms is transformed into iodide captured by thyroid and is converted thereafter into thyroid hormones, thyroxin and triiodothyronine.

Over 90% iodine is removed from the organism with urine under the form of iodide.

Actually, the therapy of thyroid tumors is first of all surgical but the surgical operation does not succeed in removing all of the thyroid tissues and for that reason a destruction of thyroid tissues is made after the intervention by selective radiation (radio ablation), as mentioned above.

In practice, after the surgical operation, therapeutic doses of ¹³¹I in the form of NaI (sodium iodide) are orally administered to the patient; iodine is partially removed with urine and partially accumulated in the remaining thyroid tissues that are irradiated selectively (iodine is beta-emitting) until the death of the residual tumoral tissue cells is caused.

The same principle of radio ablation is used in the destruction of metastases of thyroid tumors.

¹³¹I which is captured by thyroid, however, is in competition with iodide in the organism so that it is advisable to give the patient a diet with low iodine content before the treatment.

In many cases, however, a high iodine content is found in the organism with a strong inhibition effect.

The excess of iodine is often due to the use of an iodinated contrast medium used in CAT and generally in the radiographic diagnostics; in other cases, such excess is due to the use of drugs or disinfectants containing iodine.

In all cases the radio ablation therapy is relatively ineffective because of the low specific capture of ¹³¹I. For such reason it is important to carry out an accurate analytical screening of I⁻ in urine.

There is the need for all Nuclear Medicine Wards carrying out treatments with radioactive iodine to have a simple, reliable kit to make the analytical-quantitative iodine content determination in real time.

The determination of I⁻ in urine that usually is made in chemical laboratories is presently carried out mainly according to two different analytical methodologies. The first is a direct potentiometric iodide content determination by an iodine-selective electrode or iodide spectrophotometry by Sandell-Kolthoff reaction.

In the direct potentiometric determination the iodide content in urine is determined by measuring the potential of a selective electrode with respect to a reference electrode both in the urine sample and in several standard samples.

Even if such methodology guarantees sufficient reproducibility, it has a reduced sensitivity for low iodide contents. Furthermore, the selective electrode is very delicate and particularly subject to dirt and has a high cost as well as a rather short life (about six months) .

The second methodology commonly used, i.e. iodide spectrophotometry by Sandell-Kolthoff reaction, is based on the fact that iodide acts as catalyzer in the reduction of ion Ce⁴⁺→Ce³⁺ coupled to oxidation As³⁺→ As⁵⁺. Ion Ce⁴⁺ is coloured intense yellow, while ion Ce³⁺ is colourless. A spectrophotometry of the remaining Ce⁴⁺ allows the iodine content to be determined.

To sum up, arsenious acid and then cerium sulphate is added to a test-tube containing urine whose iodine content has to be determined. The intensity of yellow coloration is red at the wavelength of 405 nm and the decrease in the intensity with respect to the initial intensity is proportional to the quantity of I⁻ (iodide) in solution.

The method is rather reliable, accurate and sensitive.

Before the determination it is necessary to remove some molecules interfering with the Sandell-Kolthoff reaction such as thiocyanate (SCN⁻) which is typically present in the smokers' urine in remarkable quantity.

Such removal is obtained by heat treatment of the urine with chloric acid, or with a mixture of chloric acid and sodium chromate, or with ammonium persulphate.

A drawback of such known methodology is that it is necessary to have a good spectrophotometer for the quantitative determination of iodine. Moreover, such determination cannot be used for measurements on the field.

The need of an analytical method for the determination of iodine in urinc that is quick and cheap and that can also be used outside the analysis laboratories led the known Company MERCK® to patent and put on the market a kit called "Merck Rapid Urinary Test", where urine is passed through a column of activated carbon for removing some interfering molecules, and then tetramethylbenzidine is oxidized with peracetic acid, with iodine as catalyzer.

The urine can be divided into three groups by the colour obtained after the reaction:
- < 10 µg/dl (yellow)
- 11-30 µg/dl (green)
- > 30 µg/dl (blue).

The analysis method provided by such known kit is simple and reliable, but allows only a semiquantitative division into three groups.

The main purpose of the present invention is to overcome the problems and the drawbacks mentioned above by providing an analytical method and a simple, cheap apparatus capable of providing quantitative results of the iodide concentration in urine and other biological fluids also in laboratories without analytical instruments such as spectrophotometer and precision potentiometer.

This has been obtained according to the invention by providing a method and an apparatus that make use of the inverse proportion between the colour change time of a solution of the biological fluids to be analyzed and the iodine content in the latter, even if they are essentially based on the already mentioned Sandell-Kolthoff reaction.

A better understanding of the invention will result from the reference to the drawings that show schematically a preferred embodiment of the apparatus only by way of a not limiting example.

In the drawings:
Figure 1 is a diagram showing the calibration curve obtained from known quantities of iodine; the same diagram shows the values obtained by analyzing urine of two healthy subjects; and
Figure 2 is a schematic diagram showing an apparatus for carrying out the method according to the invention.

It is known that the reduction of Ce from +4 to +3 is the more rapid the more iodine is present in urine.

Therefore, according to the invention, the time between the addition of reagents to urine and the complete reduction of Ce⁴⁺ can be measured by adding an excess of arsenious acid, cerium sulphate, and an indicator of oxidoreduction.

It was experimentally found that such a time is inversely proportional to the content of iodide which is the catalyzer of the reaction.

Therefore, according to the invention, it is sufficient to measure the reduction times of the unknown sample and some standard samples to determine the iodine content in the sample of the analyzed human fluid.

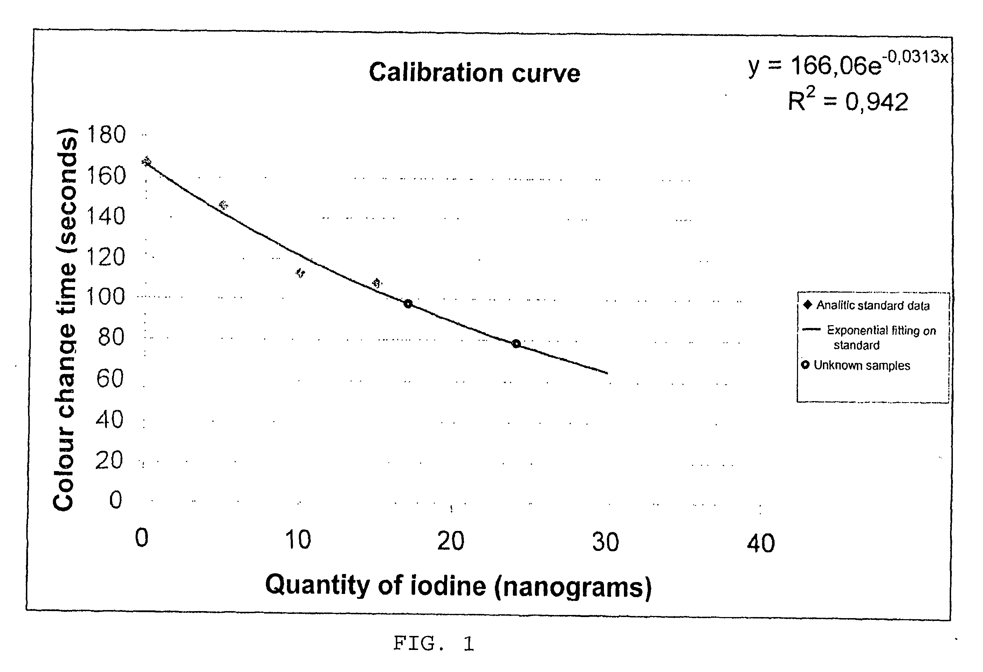

To this purpose, the method of the invention comprises essentially the following steps:
1. removing selectively some interfering substances such as thiocyanate (SCN⁻) by their oxidation with the addition of a first reagent A to a solution of the biological fluid to be analyzed;
2. heating the solution containing the biological fluid to a temperature between 100 and 110°C for about 60 minutes with next cooling;
3. adding a second reagent B able to create a strongly reducing environment in the solution;
4. adding a third reagent C able to indicate the state of oxidoreduction in the solution;
5. adding a fourth reagent D having the function of oxidizing agent to the solution, and beginning the time counting;
6. monitoring the solution colour change till the appearance of the first clearly visible, pink-orange shade, at which appearance the time from the addition of the fourth reagent D is detected and recorded;
7. determining the iodine content in the solution by reading the diagram of Figure 1 versus the detected time.

By way of illustration, an example of the determination according to the method described above is disclosed below, wherein the biological fluid is a 10+100%, prcfcrably 50%, water solution of urine.

250 µl H₂O and 500 µl reagent A, preferably consisting of 5-35%, preferably 28%, chloric acid is put into a glass test-tube; said solution is then heated to about 100-110°C for a time between 5 minutes and 2 hours, preferably 60 minutes.

Reagent A acting as oxidant has the purpose of destroy selectively some substances, especially thiocyanate, that are present particularly in the smokers' urine and strongly interfere with Sandell-Kolthoff reaction as well as the determination of iodine.

In alternative to chloric acid as oxidant, persulphate "suffering" from low reproducibility of the results can also be used.

After the solution is cooled, are added, in sequence:
- 500 µl of reducing reagent B preferably consisting of a solution of 1ö50 mg/ml, preferably 10 mg/ml, As₂O₃, and 10ö100 mg/ml, preferably 25 mg/ml, NaCl dissolved in 0.1Nö10N, preferably 1N, sulphuric acid;
- 20 µl reagent C, which is the indicator of oxidoreduction, preferably consisting of 0.1ö10%, preferably 6%, ortho-ferro-phenanthroline, and
- 20 µl reagent D, which is the oxidizing agent of the reaction, preferably consisting of 0.01Nö3N, preferably 0.2N, cerium sulphate in 0.1Nö10N, preferably 3.5N, sulphuric acid.

Upon adding reagent D, a chronometer is started. Upon adding cerium, the colour of the solution changes immediately from orange to pale green.

Reagent B creates a strongly reducing environment in the solution, while reagent C provides the indication of the oxidoreduction state in the solution. An orange colour indicates the presence of Fe²⁺ in the complex of iron and ortho-ferro-phenanthroline, i.e. a reducing environment, while a pale blue colour indicates the presence of Fe³⁺ in the complex, i.e. an oxidizing environment.

A reducing environment is created by adding reagents B and C to urine so that ortho-ferro-phenanthroline is coloured orange.

An oxidizing environment is created by the addition of reagent D so that the oxidation of iron in the ortho-ferro-phenanthroline complex which is coloured pale blue takes place quickly, while the oxidation of arsenite to arsenate by tetravalent cerium, that is cataliyzed by iodide, takes place slowly.

As the reduction reaction of Ce⁴⁺ by As³⁺ takes place, the solution is coloured pale green resulting from the presence of Ce⁴⁺ which is coloured yellow and oxidized ortho-ferro-phenanthroline which is coloured pale blue.

While Ce⁴⁺ is reduced to Ce³⁺, i.e. the yellow colour disappears, the solution is discoloured slowly until a pale blue colour appears upon consuming all of Ce⁴⁺. At that time, the excess of arsenite acting as reducing agent causes ortho-ferro-phenanthroline to turn to its original orange colour.

Therefore, upon appearing the first clearly visible pink-orange shade, the chronometer is stopped and the elapsed time is drawn on a diagram and compared with several standard times at gradually increasing iodide concentrations.

Thus, according to the invention, the simple measurement of the time of the colour change to the original orange colour allows a diagram such as shown in Figure 1 to be immediately plotted so as to provide the iodide content in the analyzed urine.

A three-time repeated determination example over three standards gave the following results:

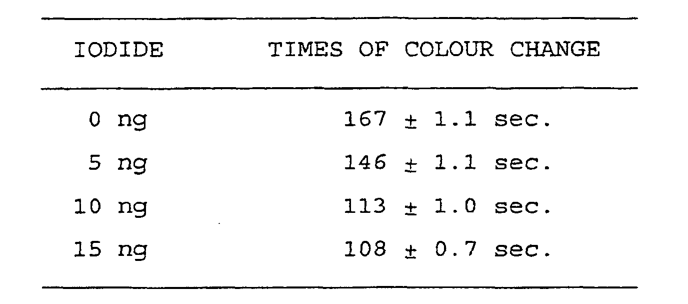

As can be seen, the reproducibility of the response is excellent.

The iodide contents found and plotted on the diagram of Figure 1 (23.7 ng and 16.5 ng) correspond to 9.5 µg/dl and 6.6 µg/dl and fall exactly in the defined typical range between 5 µg/dl and 30 µg/dl.

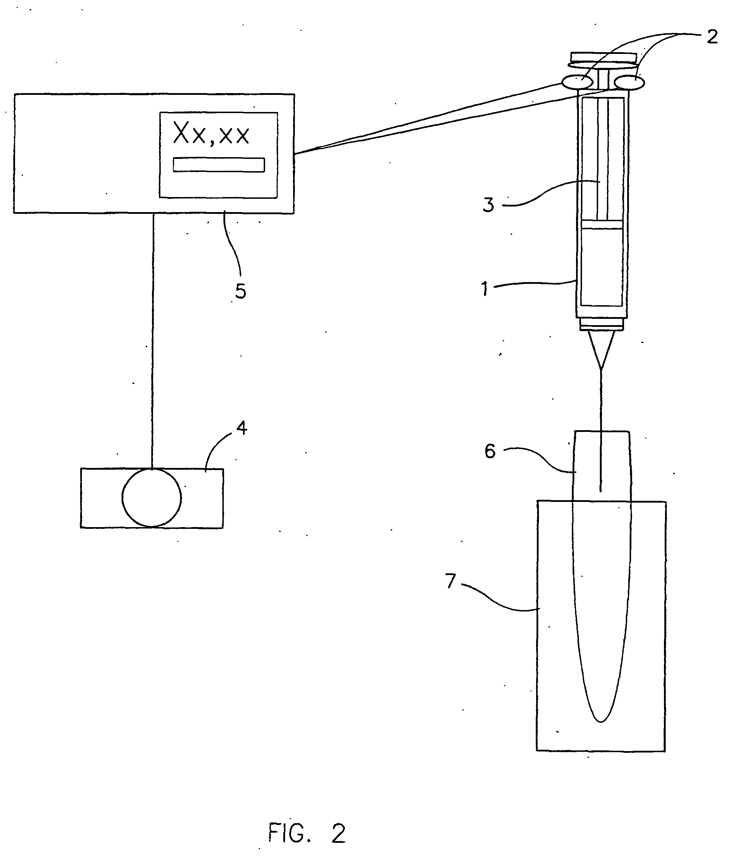

According to the present invention, in order to facilitate the execution of the disclosed method by detecting the right times of beginning and ending of the colour change, the apparatus shown in Figure 2 has been conceived.

The disclosed apparatus includes essentially:
- a test-tube 6 for the solution of the biological fluid to be analyzed;
- three syringes or pipettes (not shown) containing the right amount of reagents A, B and C to be added to the solution in the test-tube;
- a special syringe 1 provided with electrical contacts 2 for the end of stroke of piston 3 and containing the fourth reagent D to be added to the solution in the test-tube;
- a chronometer or timer 5 which is started by the closure of said electrical end-of-stroke contacts and stopped by a suitable stop button 4;
- a support 7 for test-tube 6 which is preferably lit at the back and capable of facilitating the detection of the colour change of the solution in the test-tube.

In such apparatus, each one of the first three reagents A, B and C is added to the solution of biological fluid by a common syringe or a pipette, while reagent D is added by a suitable syringe 1 provided with electrical contacts 2 of the end of stroke of piston 3, as shown in Figure 2.

After such addition, the electrical contacts 2 of syringe 1 start a preferably digital chronometer 5.

In operation, as soon as fourth reagent D is put into test-tube 6, the latter is stirred quickly and shortly, and put onto a support 7 which is preferably lit at the back so that the operator stops chronometer 5 by pressing the suitable stop button 4, as soon as the characteristic red shade caused by the reduction of the ortho phenanthroline appear.

The simple apparatus according to the invention allows an excellent reproducibility in time associated with a remarkable easiness of use.

Furthermore, other biological liquids such as plasma or protein hydrolysates can also be analyzed by such method.

The disclosed apparatus is economical and reliable due to its simple components and can be of particular interest not only for all medical laboratories which need to determine the iodide content in the urine for investigating alimentary deficiencies but also for all Nuclear Medicine Wards that can thus carry out on their own a rapid, reliable determination of the physiological iodide content and the variation of such iodide content after a therapeutic treatment of all patients destined to a thyroid radio ablation treatment.

The present invention has been described and illustrated according to a preferred embodiment thereof, however, it is self-evident that anyone skilled in the art can make equivalent modifications and/or replacements without departing from the scope of the present industrial invention.

## Claims

1. A method of determining the iodine content in biological fluids, **characterized by** the measurement of the time of complete reaction of a biological fluid solution to be analyzed, on the base of the known Sandell-Kolthoff reaction, which is ascertained by the change of colour of the same solution, and the determination of the iodine content in the solution by making use of the inverse proportion between the colour change time of a solution and the iodine content in the latter acting as catalyzer, said determination of the iodine content being carried out by standard curve diagrams plotting the iodine content versus the colour change times.

2. The method according to claim 1, **characterized in that** it includes essentially the following steps:
a) removing selectively some interfering substances such as thiocyanate (SCN⁻) by their oxidation with the addition of a first reagent (A) to a solution of the biological fluid to be analyzed;
b) heating the solution containing the biological fluid to a temperature between 100 and 110°C for a time between 15 minutes and 2 hours with next cooling;
c) adding a second reagent (B) able to create a strongly reducing environment in the solution;
d) adding a third reagent (C) able to indicate the state of oxidoreduction in the solution;
e) adding a fourth reagent (D) having the function of oxidizing agent to the solution, and beginning the time counting;
f) monitoring the solution colour changes till the appearance of the first clearly visible, pink-orange shade, at which appearance the time from the addition of the fourth reagent (D) is detected;
g) determining the iodine content in the solution by reading the curve diagram of the iodine content versus the detected time.

3. The method according to claim 1 or 2, **characterized in that** said solution of biological fluids is a 10% to 100% by volume water solution of urine.

4. The method according to claim 2, **characterized in that** the first reagent (A) is 5% to 35% chloric acid.

5. The method according to claim 2, **characterized in that** the second reducing reagent (B) is a solution of 1ö50 mg/ml As₂O₃ and 10ö100 mg/ml NaCl dissolved in 0.1Nö10N sulphuric acid.

6. The method according to claim 2, **characterized in that** the third reagent (C), an indicator of oxidoreduction, is 0.1%ö10% ortho-ferro-phenanthroline.

7. The method according to claim 2, **characterized in that** the fourth reagent (D), an oxidant of the reaction, is 0.01Nö3N cerium sulphate in 0.1Nö10N sulphuric acid.

8. The method according to the preceding claims, **characterized in that** 250 µl urine, 250 µl H₂O and 500 µl first agent (A) are put into a glass test-tube, then said solution is heated for 60 minutes at about 100ö110°C, after cooling 500 µl second reducing reagent (B), 20 µl third reagent (C), indicator of oxidoreduction, and 20 µl fourth reagent (D) which is the oxidizing agent of reaction are added and stirred shortly, wherein when adding the last reagent (D) a chronometer is started which is only stopped when the first pink-orange shade appear in the pale blue colour.

9. The method according to claim 2, **characterized in that** the heating of step b) lasts 60 minutes.

10. The method according to claim 3, **characterized in that** the water solution of urine is a 50% by volume solution.

11. The method according to claim 4, **characterized in that** first reagent (A) is 28% chloric acid.

12. The method according to claim 5, **characterized in that** second reagent (B) is a solution consisting of 10 mg/ml As₂O₃ and 35 mg/ml NaCl dissolved in 1N sulphuric acid.

13. The method according to claim 6, **characterized in that** third reagent (C) is 6% ortho-ferro-phenanthroline.

14. The method according to claim 7, **characterized in that** fourth reagent (D) is 0.2N cerium sulphate in 3.5N sulphuric acid.

15. An apparatus for determining the iodine content in biological fluids, **characterized in that** it comprises:
- a test-tube (6) for the solution of the biological fluid to be analyzed;
- three syringes or pipettes (not shown) containing the right amount of the first three reagents (A, B and C) to be added to the solution in the test-tube;
- a special syringe (1), or some similar apparatus for delivery of precise amounts of liquid, provided with electrical contacts (2) for the end of stroke of piston (3) and containing the fourth reagent (D) to be added to the solution in the test-tube;
- a chronometer or timer (5) which is started by the closure of said electrical end-of-stroke contacts (2) and stopped by a suitable stop button (4) when the solution changes colour and turns to the original colour before adding the fourth reagent;
- a support (7) for test-tube (6) which is preferably lit at the back and capable of facilitating the detection of the colour change of the solution in the test-tube.

16. The apparatus according to claim 15, **characterized in that** electrical contacts (2) of syringe (1), or some similar apparatus for delivery of precise amounts of liquid, are positioned such that they start a chronometer (5) as soon as the addition of fourth reagent (D) is ended.

17. The apparatus according to claim 15 or 16, **characterized in that** the biological fluid is urine, plasma, or protein hydrolisates.

18. The method according to any claim 1 to 14, **characterized in that** the biological fluid is urine, plasma, or protein hydrolisates.

## Patentansprüche

1. Verfahren zur Bestimmung des Jodgehaltes in biologischen Fluiden, **gekennzeichnet durch** die Messung der Zeit der vollständigen Umsetzung einer biologischen, fluiden Lösung, die analysiert werden soll, auf Basis der bekannten Sandell-Kolthoff Reaktion, die **durch** die Änderung der Farbe der Lösung ermittelt wird, und die Bestimmung des Jodgehaltes in der Lösung unter Zuhilfenahme des inversen Verhältnisses zwischen der Farbänderungszeit einer Lösung und dem Iodgehalt in letzterer, der als Katalysator wirkt, wobei die Bestimmung des Jodgehaltes **durch** Standardkurvendiagramme durchgeführt wird, in denen der Jodgehalt gegen die Farbänderungszeit aufgetragen wird.

2. Verfahren gemäß Anspruch 1, **dadurch gekennzeichnet, daß** es im wesentlichen die folgenden Schritte beinhaltet:
a) selektives Entfernen einiger störender Substanzen, wie zum Beispiel Thiocyanate (SCN⁻) durch deren Oxidation mittels Zugabe eines ersten Reagenzes (A) zu einer Lösung des biologischen Fluids, das analysiert werden soll;
b) Erhitzen der Lösung, die das biologische Fluid enthält, auf eine Temperatur zwischen 100 und 110°C für eine Zeit zwischen 15 Minuten und 2 Stunden mit anschließender Abkühlung;
c) Zugeben eines zweiten Reagenzes (B), das in der Lage ist, eine stark reduzierende Umgebung in der Lösung herzustellen;
d) Zugeben eines dritten Reagenzes (C), das in der Lage ist, den Zustand der Oxidoreduktion der Lösung anzuzeigen;
e) Zugeben eines vierten Reagenzes (D) mit der Funktion eines Oxidationsmittels zu der Lösung, und Beginn der Zeitnahme;
f) Überwachen der Farbänderungen der Lösung bis zum Erscheinen des ersten, klar sichtbaren, pink-orangen Farbtönung, bei deren Auftreten die Zeit ab der Zugabe des vierten Reagenzes (D) erfaßt wird;
g) Bestimmung des Jodgehaltes in der Lösung durch Ablesen des Kurvendiagramms des Jodgehaltes gegenüber der ermittelten Zeit.

3. Verfahren gemäß Anspruch 1 oder 2, **dadurch gekennzeichnet, daß** die Lösung des biologischen Fluids eine 10 Vol.-%-ige bis 100 Vol.-%-ige Wasserlösung von Urin ist.

4. Verfahren gemäß Anspruch 2, **dadurch gekennzeichnet, daß** das erste Reagenz (A) 5%-ige bis 35%-ige Chlorsäure ist.

5. Verfahren gemäß Anspruch 2, **dadurch gekennzeichnet, daß** das zweite Reduktionsmittel (B) eine Lösung von 1-50 mg/ml As₂O₃ und 10-100 mg/ml NaCl, aufgelöst in 0,1N-10N Schwefelsäure, ist.

6. Verfahren gemäß Anspruch 2, **dadurch gekennzeichnet, daß** das dritte Reagenz (C), ein Indikator der Oxidoreduktion, 0,1% bis 10% ortho-Ferrophenanthrolin ist.

7. Verfahren gemäß Anspruch 2, **dadurch gekennzeichnet, daß** das vierte Reagenz (D), ein Oxidationsmittel der Reaktion, 0,01N-3N Cersulfat in 0,1N bis 10N Schwefelsäure ist.

8. Verfahren gemäß den vorangegangenen Ansprüchen, **dadurch gekennzeichnet, daß** 250 µl Urin, 250 µl H₂O und 500 µl des ersten Reagenz (A) in ein Reagenzglas gegeben werden, anschließend die Lösung für 60 Minuten auf ungefähr 100 bis 110°C erhitzt wird, nach Abkühlen 500 µl des zweiten Reduktionsmittels (B), 20 µl des dritten Reagenz (C), dem Indikator der Oxidoreduktion, und 20 µl des vierten Reagenz (D), welches das Oxidationsmittel der Reaktion ist, zugegeben werden, und kurz gerührt wird, wobei bei der Zugabe des letzten Reagenz (D) ein Zeitmesser gestartet wird, der erst angehalten wird, wenn die erste pink-orange Farbtönung in der blassblauen Farbe auftritt.

9. Verfahren gemäß Anspruch 2, **dadurch gekennzeichnet, daß** die Erwärmung in Schritt b) 60 Minuten andauert.

10. Verfahren gemäß Anspruch 3, **dadurch gekennzeichnet, daß** die Wasserlösung des Urins eine 50 Vol.-%-ige Lösung ist.

11. Verfahren gemäß Anspruch 4, **dadurch gekennzeichnet, daß** das erste Reagenz (A) 28%-ige Chlorsäure ist.

12. Verfahren gemäß Anspruch 5, **dadurch gekennzeichnet, daß** das zweite Reagenz (B) eine Lösung, bestehend aus 10 mg/ml As₂O₃ und 35 mg/ml NaCl, aufgelöst in 1N Schwefelsäure, ist.

13. Verfahren gemäß Anspruch 6, **dadurch gekennzeichnet, daß** das dritte Reagenz (D) 6%-iges ortho-Ferrophenanthrolin ist.

14. Verfahren gemäß Anspruch 7, **dadurch gekennzeichnet, daß** das vierte Reagenz (D) 0,2 N Cersulfat in 3,5N Schwefelsäure ist.

15. Gerät zur Bestimmung des Jodgehaltes in biologischen Fluiden, **dadurch gekennzeichnet, daß** es folgendes umfaßt:
- ein Reagenzglas (6) für die Lösung des zu analysierenden, biologischen Fluids;
- drei Spritzen oder Pipetten (nicht gezeigt), die die richtige Menge der ersten drei Reagenzien (A, B und C), die zu der Lösung in dem Reagenzglas zugegeben werden soll, enthalten;
- eine spezielle Spritze ( 1 ), oder ein ähnliches Gerät zur Einbringung einer exakten Menge einer Flüssigkeit, ausgestattet mit elektrischen Kontakten (2) am Ende des Kolbenhubs (3) und die das vierte Reagenz (D), das zu der Lösung im Reagenzglas zugegeben werden soll, enthält;
- einen Zeitmesser oder Zeitnehmer (5), der durch das Schließen der elektrischen Kontakte am Ende des Hubs (2) gestartet wird und durch einen geeigneten Stopknopf (4) angehalten wird, wenn die Lösung die Farbe ändert und sich zu der Ausgangsfarbe ändert, bevor das vierte Reagenz zugegeben wurde;
- einen Träger (7) für das Reagenzglas (6), der bevorzugt auf der Rückseite beleuchtet ist und in der Lage ist, die Wahrnehmung der Farbänderung der Lösung im Reagenzglas zu erleichtern.

16. Gerät gemäß Anspruch 15, **dadurch gekennzeichnet, daß** die elektrischen Kontakte (2) der Spritze (1) oder eines ähnlichen Gerätes zur Einbringung der exakten Menge an Flüssigkeit, so angebracht sind, daß sie einen Zeitnehmer (5) starten, sobald die Zugabe des vierten Reagenz (D) beendet ist.

17. Gerät gemäß Anspruch 15 oder 16, **dadurch gekennzeichnet, daß** das biologische Fluid Urin, Plasma oder Proteinhydrolisate ist.

18. Verfahren gemäß einem der Ansprüche 1 bis 14, **dadurch gekennzeichnet, daß** das biologische Fluid Urin, Plasma oder Proteinhydrolisate ist.

## Revendications

1. Procédé de détermination de la teneur en iode dans des fluides biologiques, **caractérisé en ce qu'**on mesure la durée de la réaction totale d'une solution de fluide biologique à analyser, sur la base de la réaction Sandell/Kolthoff connue, qui est constatée par le changement de teinte de ladite solution, et **en ce qu'**on détermine la teneur en iode dans la solution en utilisant la proportion inverse entre le moment où la solution change et la teneur en iode de cette dernière, qui fait office de catalyseur, ladite détermination de la teneur en iode étant mise en oeuvre par le tracé de courbes schématiques standard de la teneur en iode en fonction des temps de changement de teinte.

2. Procédé selon la revendication 1, **caractérisé en ce qu'**il comprend essentiellement les étapes suivantes :
a) élimination sélective de certaines substances perturbatrices telles que le thiocyanate (SCN⁻) sous l'effet de leur oxydation en ajoutant un premier réactif (A) à une solution du fluide biologique à analyser ;
b) chauffage de la solution contenant le fluide biologique à une température comprise entre 100 et 110°C pendant une durée comprise entre 15 minutes et 2 heures avec un refroidissement consécutif ;
c) ajout d'un deuxième réactif (B) en mesure de créer un environnement fortement réducteur dans la solution ;
d) ajout d'un troisième réactif (C) en mesure d'indiquer l'état d'oxydoréduction dans la solution ;
e) ajout d'un quatrième réactif (D) faisant office d'agent oxydant à la solution, et début du décompte du temps ;
f) suivi des changements de teinte de la solution jusqu'à l'apparition de la première nuance rose - orangée, bien visible, à laquelle on détecte la durée à partir de l'ajout du quatrième réactif (D) ;
g) détermination de la teneur en iode dans la solution en lisant la courbe schématique de la teneur en iode par rapport à la durée détectée.

3. Procédé selon l'une quelconque des revendications 1 ou 2, **caractérisé en ce que** ladite solution de fluides biologiques est une solution aqueuse d'urine, de 10 % à 100 % en volume.

4. Procédé selon la revendication 2, **caractérisé en ce que** le premier réactif (A) est composé d'acide chlorique de 5 % à 35 %.

5. Procédé selon la revendication 2, **caractérisé en ce que** le deuxième réactif réducteur (B) est une solution composée de 1 à 50 mg/ml d'As₂O₃ et de 10 à 100 mg/ml de NaCl dissous dans de l'acide sulfurique 0,1N à 10N.

6. Procédé selon la revendication 2, **caractérisé en ce que** le troisième réactif (C), à savoir un indicateur d'oxydoréduction, est de l'ortho-ferrophénanthroline de 0,1 % à 10 %.

7. Procédé selon la revendication 2, **caractérisé en ce que** le quatrième réactif (D), un oxydant de la réaction, est du sulfate de cérium 0,01N à 3N dans de l'acide sulfurique 0,1 N à 10 N.

8. Procédé selon l'une quelconque des revendications précédentes, **caractérisé en ce que** 250 µl d'urine, 250 µl de H₂O et 500 µl du premier agent (A) sont introduits dans un tube à essais en verre, puis ladite solution est chauffée pendant 60 minutes à une température comprise entre environ 100 et 110° C, après refroidissement, 500 µl du deuxième agent réducteur (B), 20 µl du troisième réactif (C), à savoir l'indicateur d'oxydoréduction, et 20 µl du quatrième réactif (D), qui est l'agent oxydant de la réaction, sont ajoutés et mélangés pendant un court laps de temps, moyennant quoi, quand on ajoute le dernier réactif (D), on fait démarrer un chronomètre qui s'arrête seulement quand la première nuance rose orangée apparaît dans la teinte bleu pâle.

9. Procédé selon la revendication 2, **caractérisé en ce que** le chauffage de l'étape b) dure pendant 60 minutes.

10. Procédé selon la revendication 3, **caractérisé en ce que** la solution aqueuse d'urine est une solution à 50 % en volume.

11. Procédé selon la revendication 4, **caractérisé en ce que** le premier réactif (A) est de l'acide chlorique à 28 %.

12. Procédé selon la revendication 5, **caractérisé en ce que** le deuxième réactif (B) est une solution composée de 10 mg/ml d'As₂O₃ et de 35 mg/ml de NaCl dissous dans de l'acide sulfurique 1N.

13. Procédé selon la revendication 6, **caractérisé en ce que** le troisième réactif (C) est de l'ortho-ferrophénanthroline à 6 %.

14. Procédé selon la revendication 7, **caractérisé en ce que** le quatrième réactif (D) est du sulfate de cérium 0,2N dans de l'acide sulfurique 3,5N.

15. Appareil pour déterminer la teneur en iode dans des fluides biologiques, **caractérisé en ce qu'**il comprend :
- un tube à essais (6) pour la solution du fluide biologique à analyser ;
- trois seringues ou pipettes (non représentées) contenant la quantité appropriée des trois premiers réactifs (A, B et C) devant être ajoutés à la solution dans le tube à essais ;
- une seringue spéciale (1), ou autre appareil similaire permettant de délivrer les quantités précises de liquide, dotée de contacts électriques (2) pour la fin de course du piston (3), et contenant le quatrième réactif (D) devant être ajouté à la solution dans le tube à essais ;
- un chronomètre ou une minuterie (5) qui démarre sous l'effet de la fermeture desdits contacts de fin de course électriques (2) et s'arrête grâce à un bouton (4) approprié quand la teinte de la solution change et revient à la couleur initiale avant l'addition du quatrième réactif ;
- un support (7) pour le tube à essais (6), qui est de préférence éclairé à l'arrière et est en mesure de faciliter la détection du changement de teinte de la solution dans le tube à essais.

16. Appareil selon la revendication 15, **caractérisé en ce que** les contacts électriques (2) de la seringue (1), ou autre appareil similaire permettant de délivrer des quantités précises de liquide, sont positionnés de telle sorte qu'ils font démarrer un chronomètre (5) dès que l'addition du quatrième réactif (D) est terminée.

17. Appareil selon l'une quelconque des revendications 15 ou 16, **caractérisé en ce que** le fluide biologique est de l'urine, du plasma ou des hydrolisats de protéines.

18. Procédé selon l'une quelconque des revendications 1 à 14, **caractérisé en ce que** le fluide biologique est de l'urine, du plasma ou des hydrolisats de protéines.
